# EUROPEAN PATENT APPLICATION

(11) **EP 4 674 278 A1**
(43) Date of publication of application: **07.01.2026**
(21) Application number: 24764155.8
(22) Date of filing: 27.02.2024
(51) Int. Cl.: A23K 10/16, A23K 50/80, C12R 1/89

(54) **METHOD FOR MANUFACTURING SALMON AQUACULTURE FEED USING NUTRIENT-RICH MICROALGAE SCHIZOCHYTRIUM SP. BIOMASS**

(30) Priority: 28.02.2023 KR 20230027303
(71) Applicant: CJ CheilJedang Corporation, Seoul 04560 (KR)
(72) Inventor: LEE, Young Mi, Seoul 04560 (KR); CHO, Kookjin, Seoul 04560 (KR); LIM, Eu Gene, Seoul 04560 (KR); YU, Seongheun, Seoul 04560 (KR); SEO, Sang Hyun, Seoul 04560 (KR); KIM, Ji Young, Seoul 04560 (KR)
(74) Representative: Meissner Bolte Partnerschaft mbB
(86) International application number: PCT/KR2024/002470
(87) International publication number: WO 2024/181756

(57) **Abstract**

A feed composition comprising microalgae-derived biomass according to the present application uses microalgae having a high content of protein, fat, and minerals and free of factors that degrade feed quality, and thus can be effectively used as a fish feed composition.

## Description

### TECHNICAL FIELD

### Cross-reference to related application(s)

The present application claims benefit of priority to Republic of Korea Patent Application No. 10-2023-0027303, filed on February 28, 2023, the entire content of which are incorporated herein by reference.

The present application relates to a feed composition for fish of family Salmonidae comprising a biomass derived from microalgae and a manufacturing method thereof.

### BACKGROUND ART

With global population growth and stagnation and continued decline in grain and fishery production, concerns about future food security persist, and many scientists assume that an aquaculture industry has the only potential to solve these food problems. A world cultivated fish production started from 2.4 million tons a half century ago and reached 87.5 million tons in 2020, which accounts for 49% of the total fishery production, feed consumption of marine fish and shellfish among these cultivated fish is growing high (FAO 2022), and this trend of increase is expected to be continued. Today, a nutrition field of fish-culture feed is one of the key factors in a success of the aquaculture industry. In the aquaculture industry, it is a well-known fact that breeding management using a high-quality feed should be supported when seed production technology is developed by first selecting important fish species that may be aquacultured. In particular, the importance of feed nutrition may be known from a fact that a feed cost accounts for 30-60% of an aquaculture production cost, although the feed cost varies depending on fish species.

Feed utilization rate varies depending on a protein raw material. Fish meal is made into powder by drying fish scraps or fish crumbs left after squeezing fish oil from fish. It has long been used as a main protein source for fish-culture feed due to its high protein content, excellent amino acid composition, and high palatability. However, as the production of the fish meal has recently declined rapidly, supply is unstable and prices have soared, accounting for the largest portion of the cost of purchasing a feed raw material. The catch production of various fish that make fish meal is rapidly decreasing due to overfishing and marine environmental issues, and it is predicted that the production thereof is declined sustainably until the year 2030. For this reason, many studies are being conducted to find inexpensive and high-quality alternative protein sources that may replace the fish meal in a mixed feed for fish-culture.

### PRIOR ART DOCUMENTS

(Patent document 1) US 2015-0208696 A1

### DISCLOSURE

### TECHNICAL PROBLEM

The object of the present application is directed to providing a feed composition for fish of family Salmonidae comprising a biomass derived from microalgae.

Another object of the present application is directed to providing a method of manufacturing the feed composition.

Still another object of the present application is directed to providing a method of aquaculture for fish of family Salmonidae comprising feeding of the feed composition to the fish of family Salmonidae.

### TECHNICAL SOLUTION

An aspect provides a feed composition for fish of family Salmonidae comprising a biomass derived from microalgae, a use of improving digestibility of fish of family Salmonidae of the biomass derived from microalgae or the feed composition for fish of family Salmonidae comprising the biomass derived from microalgae, or a composition for improving digestibility of the fish of family Salmonidae comprising the biomass derived from microalgae or the feed composition for fish of family Salmonidae comprising the biomass derived from microalgae.

### Biomass derived from microalgae

In the present specification, a microalgae may be a microalgae of genus Schizochytrium.

In the present specification, the "microalgae" may refer to an organism that lives floating freely in water in plants photosynthesizing with chlorophyll, which may not be easily seen with naked eye, and thus it may be seen through a microscope and is also called phytoplankton.

In the present specification, the microalgae of genus Schizochytrium may be a microorganism belonging to the family of Thraustochytriaceae, and may be at least one selected from a group consisting of *Schizochytrium aggregatum, Schizochytrium limacinum, Schizochytrium minutum,* etc., but is not limited thereto.

In an example, the microalgae of genus Schizochytrium may be *Schizochytrium aggregatum.*

In an example, the microalgae of genus Schizochytrium may be *Schizochytrium limacinum.*

In an example, the microalgae of genus Schizochytrium may be *Schizochytrium minutum.*

In an example, the microalgae of genus Schizochytrium may be Schizochytrium sp. CD01-5004 deposited under accession number of KCTC14345BP.

In the present specification, biomass refers to organisms such as plants, animals, and microorganisms that may be used as chemical energy, that is, an energy source of bioenergy. In ecological terms, it also refers to weight or energy amount of a specific organism existing within a unit of time and space. In addition, the biomass may comprise a compound secreted by a cell, but is not limited thereto, and may contain not only extracellular materials but also cells and/or intracellular contents.

In the present specification, a biomass derived from microalgae may be the microalgae itself, a culture thereof, a fermented product thereof, a dried product thereof, a pulverized product thereof, or a product produced by culturing or fermenting the microalgae, or may be a concentrate of the biomass or the dried product, but is not limited thereto. That is, the biomass derived from microalgae may comprise at least one selected from a group consisting of the microalgae, the culture of the microalgae, the dried product of the culture, and the pulverized product of the dried product.

The "culture" of the microalgae refers to a product produced by culturing the microalgae, and may specifically be a culture solution comprising the microalgae or a culture filtrate from the culture solution from which the microalgae is removed, but is not limited thereto. The "dried product" of the microalgae culture is the microalgae culture from which moisture is removed and for example, may be a dried microbial cell form of the microalgae, but is not limited thereto. In addition, the "pulverized product" of the dried product collectively refers to a resulting product of pulverizing the dried product from removing moisture from the microalgae culture and for example, may be dried microbial cell powder, but is not limited thereto. The culture of the microalgae may be manufactured by inoculating the microalgae into a microalgae culture medium and according to a culturing method known in the art, and the dried product of the culture and the pulverized product thereof may also be manufactured according to a treatment or drying method of the microalgae or the culture solution known in the art.

The biomass derived from microalgae is rich in nutrients such as protein, fat, amino acid, fatty acid, and minerals comprising phosphorus, iron, copper, manganese, zinc, etc. and may be usefully used as a feed composition. In particular, the biomass derived from microalgae has a high content of essential amino acid for fish and comprises a high content of high-quality protein raw material, and thus it may be usefully used in the feed composition for fish that has a high proportion of protein in the feed.

The amino acid may comprise proteinogenic amino acids such as arginine, histidine, lysine, aspartic acid, glutamic acid, serine, threonine, asparagine, glutamine, cysteine, glycine, proline, alanine, valine, isoleucine, leucine, methionine, phenylalanine, tyrosine, tryptophan, and non-proteinogenic amino acids such as hydroxyproline, ornithine, cysteine/cystine.

In the present specification, the essential amino acid generally refers to an amino acid that may not be synthesized from precursors in a body of an organism, and in a case of fish, the essential amino acid may comprise isoleucine, leucine, valine, threonine, methionine, tryptophan, phenylalanine, alanine, arginine, and lysine. The feed composition of the present application comprises a large amount of the essential amino acid that are deficient in fish, thereby effectively supplying the essential amino acid that are deficient in fish.

In the present specification, ash may refer to ash produced by burning a sample or a sum of inorganic substances comprised in the sample. The term ash may be used interchangeably with terms such as mineral, inorganic substance, inorganic salt, and mineral substance, and may comprise one or more inorganic element selected from a group consisting of calcium, phosphorus, potassium, sulfur, sodium, chlorine, magnesium, iron, copper, manganese, iodine, cobalt, zinc, molybdenum, selenium, chromium, fluorine, boron, arsenic, tin, silicon, vanadium, and nickel.

In the present specification, the fatty acid may refer to a carboxylic acid comprising 4 to 36 carbon atoms (e.g., 8, 10, 12, 14, 16, 18, 20, 22, 24, 26, etc.) as a component of fat, and may refer to a saturated fatty acid without a double bond between carbons and an unsaturated fatty acid with the double bond. The unsaturated fatty acid may be a trans unsaturated fatty acid or a cis unsaturated fatty acid depending on an arrangement of the double bond, and may be a monounsaturated fatty acid or a polyunsaturated fatty acid depending on a number of the double bonds.

As is widely known in the art, the fatty acids may be indicated as their trivial names, IUPAC nomenclature, delta-x nomenclature, n-x nomenclature (or omega-x nomenclature), lipid number nomenclature (C:D, where C is a number of carbon atoms in the fatty acid and D is a number of double bonds in the fatty acid), etc.

In an example, the saturated fatty acid may be Caprylic acid (Octanoic acid, C8:0), Decanoic acid (Capric acid, C10:0), Lauric acid (Dodecanoic acid, C12:0), Myristic acid (Tetradecanoic acid, C14:0), Palmitic acid (Hexadecanoic acid, C16:0), Stearic acid (Octadecanoic acid, C18:0), Arachidic acid (Icosanoic acid, C20:0), Behenic acid (Docosanoic acid, C22:0), Lignoceric acid (Tetracosanoic acid, C24:0), Cerotic acid (Hexacosanoic acid, C26:0), etc., but is not limited thereto.

The unsaturated fatty acid may be Myristoleic acid ((9Z)-Tetradec-9-enoic acid, C14:1 n-5), Palmitoleic acid ((9Z)-Hexadec-9-enoic acid, C16:1 n-7), Sapienic acid ((6Z)-Hexadec-6-enoic acid, C16:1 n-10), Oleic acid ((9Z)-Octadec-9-enoic acid, C18:1 n-9), Elaidic acid ((E)-octadec-9-enoic acid, C18:1 n-9), Vaccenic acid ((11E)-Octadec-11-enoic acid, C18:1 n-7), Linoleic acid ((9Z,12Z)-Octadeca-9,12-dienoic acid, C18:2 n-6), Linolelaidic acid ((9E,12E)-Octadeca-9,12-dienoic acid, C18:2 n-6), alpha-Linolenic acid ((9Z,12Z,15Z)-Octadeca-9,12,15-trienoic acid, C18:3 n-3), Stearidonic acid ((6Z,9Z,12Z,15Z)-Octadeca-6,9,12,15-tetraenoic acid, C18:4 n-3), Arachidonic acid ((5Z,8Z,11Z,14Z)-Icosa-5,8,11,14-tetraenoic acid, C20:4 n-6), Eicosatetraenoic acid (icosa-8,11,14,17-tetraenoic acid, C20:4 n-3), Eicosapentaenoic acid ((5Z,8Z,11Z,14Z,17Z)-Icosa-5,8,11,14,17-pentaenoic acid, C20:5 n-3), Heneicosapentaenoic acid ((6Z,9Z,12Z,15Z,18Z)-henicosa-6,9,12,15,18-pentaenoic acid, C21:5 n-3), Cetoleic acid ((Z)-docos-11-enoic acid, C22:1 n-11), Erucic acid ((13Z)-Docos-13-enoic acid, C22:1 n-9), 15-docosenoic acid ((E)-docos-15-enoic acid, C22:1 n-7), Docosapentaenoic acid ((7Z,10Z,13Z,16Z,19Z)-Docosa-7,10,13,16,19-pentaenoic acid, C22:5 n-3), Docosahexaenoic acid ((4Z,7Z,10Z,13Z,16Z,19Z)-Docosa-4,7,10,13,16,19-hexaenoic acid, C22:6 n-3), Nervonic acid ((Z)-Tetracos-15-enoic acid, C24:1 n-9), etc., but is not limited thereto.

The unsaturated fatty acid may be an omega-3 fatty acid, an omega-6 fatty acid, an omega-7 fatty acid, or an omega-9 fatty acid. The omega-3 fatty acid, the omega-6 fatty acid, the omega-7 fatty acid, or the omega-9 fatty acid may be exemplified in Table 1 below, but are not limited thereto.

**[Table 1]**

| Omega fatty acid | Trivial name | Name | IUPAC name |
|---|---|---|---|
| Omega-3 fatty acid | Hexadecatrienoic acid (HTA) | 16:3 (n-3) | all-cis-7,10,13-hexadecatrienoic acid |
| Omega-3 fatty acid | α-Linolenic acid (ALA) | 18:3 (n-3) | all-cis-9,12,15-octadecatrienoic acid |
| Omega-3 fatty acid | Stearidonic acid (SDA) | 18:4 (n-3) | all-cis-6,9,12,15-octadecatetraenoic acid |
| Omega-3 fatty acid | Eicosatrienoic acid (ETE) | 20:3 (n-3) | all-cis-11,14,17-eicosatrienoic acid |
| Omega-3 fatty acid | Eicosatetraenoic acid (ETA) | 20:4 (n-3) | all-cis-8,11,14,17-eicosatetraenoic acid |
| Omega-3 fatty acid | Eicosapentaenoic acid (EPA) | 20:5 (n-3) | all-cis-5,8,11,14,17-eicosapentaenoic acid |
| Omega-3 fatty acid | Heneicosapentaenoic acid (HPA) | 21:5 (n-3) | all-cis-6,9,12,15,18-heneicosapentaenoic acid |
| Omega-3 fatty acid | Docosapentaenoic acid (DPA), Clupanodonic acid | 22:5 (n-3) | all-cis-7,10,13,16,19-docosapentaenoic acid |
| Omega-3 fatty acid | Docosahexaenoic acid (DHA) | 22:6 (n-3) | all-cis-4,7,10,13,16,19-docosahexaenoic acid |
| Omega-3 fatty acid | Tetracosapentaenoic acid | 24:5 (n-3) | all-cis-9,12,15,18,21-tetracosapentaenoic acid |
| Omega-3 fatty acid | Tetracosahexaenoic acid (Nisinic acid) | 24:6 (n-3) | all-cis-6,9,12,15,18,21-tetracosahexaenoic acid |
| Omega-6 fatty acid | Linoleic acid (LA) | 18:2 (n-6) | all-cis-9,12-octadecadienoic acid |
| Omega-6 fatty acid | Gamma-linolenic acid (GLA) | 18:3 (n-6) | all-cis-6,9,12-octadecatrienoic acid |
| Omega-6 fatty acid | Calendic acid | 18:3 (n-6) | 8E,10E,12Z-octadecatrienoic acid |
| Omega-6 fatty acid | Eicosadienoic acid | 20:2 (n-6) | all-cis-11,14-eicosadienoic acid |
| Omega-6 fatty acid | Dihomo-gamma-linolenic acid (DGLA) | 20:3 (n-6) | all-cis-8,11,14-eicosatrienoic acid |
| Omega-6 fatty acid | Arachidonic acid (AA, ARA) | 20:4 (n-6) | all-cis-5,8,11,14-eicosatetraenoic acid |
| Omega-6 fatty acid | Docosadienoic acid | 22:2 (n-6) | all-cis-13,16-docosadienoic acid |
| Omega-6 fatty acid | Adrenic acid | 22:4 (n-6) | all-cis-7,10,13,16-docosatetraenoic acid |
| Omega-6 fatty acid | Osbond acid | 22:5 (n-6) | all-cis-4,7,10,13,16-docosapentaenoic acid |
| Omega-6 fatty acid | Tetracosatetraenoic acid | 24:4 (n-6) | all-cis-9,12,15,18-tetracosatetraenoic acid |
| Omeqa-6 fatty | Tetracosapentaenoic acid | 24:5 (n-6) | all-cis-6,9,12,15,18- |
| acid | | | tetracosapentaenoic acid |
| Omega-7 fatty acid | - | 12:1 (n-7) | 5-Dodecenoic acid |
| Omega-7 fatty acid | - | 14:1 (n-7) | 7-Tetradecenoic acid |
| Omega-7 fatty acid | Palmitoleic acid | 16:1 (n-7) | 9-Hexadecenoic acid |
| Omega-7 fatty acid | Vaccenic acid | 18:1 (n-7) | 11-Octadecenoic acid |
| Omega-7 fatty acid | Rumenic acid | 18:2 (n-7) | Octadeca-9,11-dienoic acid |
| Omega-7 fatty acid | Paullinic acid | 20:1 (n-7) | 13-Eicosenoic acid |
| Omega-7 fatty acid | - | 22:1 (n-7) | 15-Docosenoic acid |
| Omega-7 fatty acid | - | 24:1 (n-7) | 17-Tetracosenoic acid |
| Omega-9 fatty acid | hypogeic acid | 16:1 (n-9) | (Z)-hexadec-7-enoic acid |
| Omega-9 fatty acid | oleic acid | 18:1 (n-9) | (Z)-octadec-9-enoic acid |
| Omega-9 fatty acid | elaidic acid | 18:1 (n-9) | (E)-octadec-9-enoic acid |
| Omega-9 fatty acid | gondoic acid | 20:1 (n-9) | (Z)-eicos-11-enoic acid |
| Omega-9 fatty acid | Mead acid | 20:3 (n-9) | (5Z,8Z,11Z)-eicosa-5,8,11-trienoic acid |
| Omega-9 fatty acid | erucic acid | 22:1 (n-9) | (Z)-docos-13-enoic acid |
| Omega-9 fatty acid | nervonic acid | 24:1 (n-9) | (Z)-tetracos-15-enoic acid |
| Omega-9 fatty acid | ximenic acid | 26:1 (n-9) | - |

The biomass derived from microalgae may comprise the protein (e.g., crude protein) in a content of 50 wt.% or more, 51 wt.% or more, 52 wt.% or more, 53 wt.% or more, 54 wt.% or more, 55 wt.% or more, 56 wt.% or more, 57 wt.% or more, 58 wt.% or more, 59 wt.% or more, 60 wt.% or more, 61 wt.% or more, 62 wt.% or more, 63 wt.% or more, 64 wt.% or more, 65 wt.% or more, 66 wt.% or more, 67 wt.% or more, 68 wt.% or more, 69 wt.% or more, 70 wt.% or more, 71 wt.% or more, 72 wt.% or more, 73 wt.% or more, 74 wt.% or more, 75 wt.% or more, 50 to 100 wt.%, 50 to 98 wt.%, 50 to 95 wt.%, 50 to 92 wt.%, 50 to 90 wt.%, 50 to 88 wt.%, 50 to 85 wt.%, 50 to 82 wt.%, 50 to 80 wt.%, 50 to 78 wt.%, 50 to 75 wt.%, 50 to 74 wt.%, 52 to 100 wt.%, 52 to 98 wt.%, 52 to 95 wt.%, 52 to 92 wt.%, 52 to 90 wt.%, 52 to 88 wt.%, 52 to 85 wt.%, 52 to 82 wt.%, 52 to 80 wt.%, 52 to 78 wt.%, 52 to 75 wt.%, 52 to 74 wt.%, 55 to 100 wt.%, 55 to 98 wt.%, 55 to 95 wt.%, 55 to 92 wt.%, 55 to 90 wt.%, 55 to 88 wt.%, 55 to 85 wt.%, 55 to 82 wt.%, 55 to 80 wt.%, 55 to 78 wt.%, 55 to 75 wt.%, 55 to 74 wt.%, 57 to 100 wt.%, 57 to 98 wt.%, 57 to 95 wt.%, 57 to 92 wt.%, 57 to 90 wt.%, 57 to 88 wt.%, 57 to 85 wt.%, 57 to 82 wt.%, 57 to 80 wt.%, 57 to 78 wt.%, 57 to 75 wt.%, 57 to 74 wt.%, 60 to 100 wt.%, 60 to 98 wt.%, 60 to 95 wt.%, 60 to 92 wt.%, 60 to 90 wt.%, 60 to 88 wt.%, 60 to 85 wt.%, 60 to 82 wt.%, 60 to 80 wt.%, 60 to 78 wt.%, 60 to 75 wt.%, 60 to 74 wt.%, 62 to 100 wt.%, 62 to 98 wt.%, 62 to 95 wt.%, 62 to 92 wt.%, 62 to 90 wt.%, 62 to 88 wt.%, 62 to 85 wt.%, 62 to 82 wt.%, 62 to 80 wt.%, 62 to 78 wt.%, 62 to 75 wt.%, 62 to 74 wt.%, 63 to 100 wt.%, 63 to 98 wt.%, 63 to 95 wt.%, 63 to 92 wt.%, 63 to 90 wt.%, 63 to 88 wt.%, 63 to 85 wt.%, 63 to 82 wt.%, 63 to 80 wt.%, 63 to 78 wt.%, 63 to 75 wt.%, 63 to 74 wt.%, 64 to 100 wt.%, 64 to 98 wt.%, 64 to 95 wt.%, 64 to 92 wt.%, 64 to 90 wt.%, 64 to 88 wt.%, 64 to 85 wt.%, 64 to 82 wt.%, 64 to 80 wt.%, 64 to 78 wt.%, 64 to 75 wt.%, 64 to 74 wt.%, 65 to 100 wt.%, 65 to 98 wt.%, 65 to 95 wt.%, 65 to 92 wt.%, 65 to 90 wt.%, 65 to 88 wt.%, 65 to 85 wt.%, 65 to 82 wt.%, 65 to 80 wt.%, 65 to 78 wt.%, 65 to 75 wt.%, 65 to 74 wt.%, 66 to 100 wt.%, 66 to 98 wt.%, 66 to 95 wt.%, 66 to 92 wt.%, 66 to 90 wt.%, 66 to 88 wt.%, 66 to 85 wt.%, 66 to 82 wt.%, 66 to 80 wt.%, 66 to 78 wt.%, 66 to 75 wt.%, 66 to 74 wt.%, 66 to 100 wt.%, 66 to 98 wt.%, 66 to 95 wt.%, 66 to 92 wt.%, 66 to 90 wt.%, 66 to 88 wt.%, 66 to 85 wt.%, 66 to 82 wt.%, 66 to 80 wt.%, 66 to 78 wt.%, 66 to 75 wt.%, 66 to 74 wt.%, 68 to 100 wt.%, 68 to 98 wt.%, 68 to 95 wt.%, 68 to 92 wt.%, 68 to 90 wt.%, 68 to 88 wt.%, 68 to 85 wt.%, 68 to 82 wt.%, 68 to 80 wt.%, 68 to 78 wt.%, 68 to 75 wt.%, 68 to 74 wt.%, 70 to 100 wt.%, 70 to 98 wt.%, 70 to 95 wt.%, 70 to 92 wt.%, 70 to 90 wt.%, 70 to 88 wt.%, 70 to 85 wt.%, 70 to 82 wt.%, 70 to 80 wt.%, 70 to 78 wt.%, 70 to 75 wt.%, or 70 to 74 wt.%, but is not limited thereto.

The biomass derived from microalgae may comprise the ash in a content of 1 to 20 wt.%, 1 to 15 wt.%, 1 to 10 wt.%, 2 to 20 wt.%, 2 to 15 wt.%, 2 to 10 wt.%, 3 to 20 wt.%, 3 to 15 wt.%, 3 to 10 wt.%, 4 to 20 wt.%, 4 to 15 wt.%, 4 to 10 wt.%, 5 to 20 wt.%, 5 to 15 wt.%, 5 to 10 wt.%, 6 to 20 wt.%, 6 to 15 wt.%, 6 to 10 wt.%, 7 to 20 wt.%, 7 to 15 wt.%, or 7 to 10 wt.%, but is not limited thereto.

The biomass derived from microalgae may comprise the moisture in a content of 1 to 10 wt.%, 1 to 8 wt.%, 1 to 6 wt.%, 1 to 5 wt.%, 2 to 10 wt.%, 2 to 8 wt.%, 2 to 6 wt.%, 2 to 5 wt.%, 3 to 10 wt.%, 3 to 8 wt.%, 3 to 6 wt.%, or 3 to 5 wt.%, but is not limited thereto.

The biomass derived from microalgae may comprise the fat in a content of 1 to 20 wt.%, 1 to 17 wt.%, 1 to 15 wt.%, 1 to 13 wt.%, 2 to 20 wt.%, 2 to 17 wt.%, 2 to 15 wt.%, 2 to 13 wt.%, 3 to 20 wt.%, 3 to 17 wt.%, 3 to 15 wt.%, 3 to 13 wt.%, 4 to 20 wt.%, 4 to 17 wt.%, 4 to 15 wt.%, 4 to 13 wt.%, 5 to 20 wt.%, 5 to 17 wt.%, 5 to 15 wt.%, 5 to 13 wt.%, 6 to 20 wt.%, 6 to 17 wt.%, 6 to 15 wt.%, 6 to 13 wt.%, 7 to 20 wt.%, 7 to 17 wt.%, 7 to 15 wt.%, 7 to 13 wt.%, 8 to 20 wt.%, 8 to 17 wt.%, 8 to 15 wt.%, 8 to 13 wt.%, 9 to 20 wt.%, 9 to 17 wt.%, 9 to 15 wt.%, 9 to 13 wt.%, 10 to 20 wt.%, 10 to 17 wt.%, 10 to 15 wt.%, or 10 to 13 wt.%, but is not limited thereto.

The biomass derived from microalgae may comprise the phosphorous in a content of 0.1 to 2 wt.%, 0.1 to 1.5 wt.%, 0.1 to 1.0 wt.%, 0.1 to 0.8 wt.%, 0.2 to 2 wt.%, 0.2 to 1.5 wt.%, 0.2 to 1.0 wt.%, 0.2 to 0.8 wt.%, 0.3 to 2 wt.%, 0.3 to 1.5 wt.%, 0.3 to 1.0 wt.%, or 0.3 to 0.8 wt.%, but is not limited thereto.

The biomass derived from microalgae may comprise the saturated fatty acid in a content of 10 wt. parts or more, 12 wt. parts or more, 15 wt. parts or more, 17 wt. parts or more, 20 wt. parts or more, 22 wt. parts or more, 25 wt. parts or more, 10 to 30 wt. parts, 10 to 28 wt. parts, 12 to 30 wt. parts, 12 to 28 wt. parts, 14 to 30 wt. parts, 14 to 28 wt. parts, 16 to 30 wt. parts, 16 to 28 wt. parts, 20 to 30 wt. parts, 20 to 28 wt. parts, 25 to 30 wt. parts, or 25 to 28 wt. parts based on 100 wt. parts of lipid, but is not limited thereto.

The biomass derived from microalgae may comprise the unsaturated fatty acid (e.g., omega-3 fatty acid (n-3 fatty acid or ω-3 fatty acid), or the eicosapentaenoic acid (EPA) and the docosahexaenoic acid (DHA)) in a content of 10 wt. parts or more, 15 wt. parts or more, 20 wt. parts or more, 25 wt. parts or more, 30 wt. parts or more, 35 wt. parts or more, 10 to 50 wt. parts, 10 to 48 wt. parts, 10 to 45 wt. parts, 10 to 42 wt. parts, 10 to 40 wt. parts, 10 to 38 wt. parts, 10 to 35 wt. parts, 15 to 50 wt. parts, 15 to 48 wt. parts, 15 to 45 wt. parts, 15 to 42 wt. parts, 15 to 40 wt. parts, 15 to 38 wt. parts, 15 to 35 wt. parts, 20 to 50 wt. parts, 20 to 48 wt. parts, 20 to 45 wt. parts, 20 to 42 wt. parts, 20 to 40 wt. parts, 20 to 38 wt. parts, 20 to 35 wt. parts, 25 to 50 wt. parts, 25 to 48 wt. parts, 25 to 45 wt. parts, 25 to 42 wt. parts, 25 to 40 wt. parts, 25 to 38 wt. parts, 25 to 35 wt. parts, 30 to 50 wt. parts, 30 to 48 wt. parts, 30 to 45 wt. parts, 30 to 42 wt. parts, 30 to 40 wt. parts, 30 to 38 wt. parts, or 30 to 35 wt. parts based on 100 wt. parts of lipid, but is not limited thereto.

### Feed composition

In the present specification, the feed composition may refer to something that is nutritious to animals (comprising livestock, fish, etc.) or necessary for maintaining health or growth thereof. The feed composition may be an ingredient feed, a mixed feed, or a supplementary feed. The ingredient feed may refer to a plant, animal or mineral substance used directly as a feed or as a raw material for the mixed feed. The supplementary feed may refer to that it is added to the feed to prevent deterioration of the feed quality or to increase utility of the feed. The mixed feed may be a mixture or a processing of the ingredient feed, the supplementary feed, etc. in an appropriate proportion.

The feed composition may refer to a substance that supplies organic or inorganic nutrient necessary for sustaining animal life or producing meat, milk, etc. The feed composition may additionally comprise the nutrient necessary for sustaining animal life or for producing meat, milk, etc., or any suitable excipients commonly used in the feed composition (e.g., a preservative, a wetting agent, a dispersing agent, a suspending agent, a buffer, a stabilizer, or an isotonic agent, etc.), water, a solvent, etc. The feed composition may be manufactured into various types of feed known in the art, and specifically, may comprise a concentrated feed, roughage, and/or special feed. Alternatively, the feed composition may be manufactured in a form of the mixed feed (Extruded pellet, EP feed, dry feed) or a raw feed (Moist pellet, MP feed, wet feed).

In the present specification, a feed additive may comprise substances added to a feed for various purposes such as supplementing nutrients and preventing weight loss, improving digestion availability of fiber in a feed, improving milk quality, preventing reproductive disorders and improving pregnancy rate, and preventing heat stress in summer. The feed additive may refer to the supplementary feed under control of the Livestock and Fish Feed Act, and may additionally comprise a mineral substance formulation such as sodium bicarbonate, bentonite, magnesium oxide, and a complex mineral, a mineral preparation of a trace mineral such as zinc, copper, cobalt, and selenium, a vitamin such as carotene, vitamin E, vitamin A, D, E, nicotinic acid, and vitamin B complex, an amino acid protective agent such as methionine and lysine, a fatty acid protective agent such as fatty acid calcium salt, and live bacteria and yeast such as probiotics (lactic acid bacteria), yeast culture, and fermented fungus.

The feed composition may further comprise a grain such as crushed or pulverized wheat, oat, barley, corn, and rice, a plant-based protein feed such as a feed mainly composed of a bean and sunflower, animal-based protein feed such as blood meal, meat meal, bone meal, and fish meal, sugar and dairy products such as a dry component composed of various milk powders and whey powders, and still further comprise a nutrition supplement, a digestion and absorption enhancer, a growth promoter, etc.

The feed composition may be administered to an animal alone or in combination with other feed additives among edible carriers. In addition, the feed composition may be easily administered to an animal as a top-dressing or by mixing the same directly with the feed or as an oral formulation separate from the feed. When the composition is administered separately from the feed, it may be manufactured as an immediate-release or a sustained-release formulation in combination with the edible carrier acceptable in the field of feed as is well known in the art. The edible carrier may be a solid or a liquid, for example, corn starch, lactose, sucrose, bean flake, peanut oil, olive oil, sesame oil, and propylene glycol. When a solid carrier is used, the feed composition may be a tablet, a capsule, a powder, a troche or a lozenge, or a top-dressing in a non-dispersed form. When a liquid carrier is used, the feed composition may be in a form of a soft gelatin capsule, a syrup or a suspension, an emulsion, or a solution.

The feed composition may comprise, for example, a preservative, a stabilizer, a wetting agent or an emulsifier, a cryoprotectant, or an excipient. The cryoprotectant may be at least one selected from a group consisting of glycerol, trehalose, maltodextrin, skim milk powder, and starch. The preservative, the stabilizer, or the excipient may be comprised in the composition in an effective amount sufficient to reduce deterioration of the microalgae comprised in the feed composition. In addition, the cryoprotectant may be comprised in the composition in an effective amount sufficient to reduce the deterioration of the microalgae comprised in the composition when the composition is in a dried state.

The feed composition may be used in addition to animal feed by sedimentation, spraying, or mixing.

The feed composition may be applied to a plurality of animal diets comprising mammals, birds, fish, shellfish, cephalopods, reptiles, and amphibians, but is not limited thereto. For example, the mammals may comprise a pig, a cow, a sheep, a goat, a laboratory rodent, or a pet, the birds may comprise a poultry, and the poultry may comprise a chicken, a turkey, a duck, a goose, a pheasant, or a quail, but are not limited thereto. The shellfish may comprise a shrimp, a clam, etc., but is not limited thereto. In addition, the feed composition may also be applied to a diet of a rotifer which is an animal plankton. In addition, the fish may comprise freshwater fish, saltwater fish, commercially farmed fish and a fry thereof, ornamental fish, etc.

In an example, the feed composition may be a feed composition for fish of family Salmonidae.

In the present specification, fish of family Salmonidae may refer to one or more species of fish selected from a group consisting of genus of Coregonus, genus of Prosopium, genus of Stenodus, genus of Thymallus, genus of Salvelinus, genus of Oncorhynchus, genus of Brachymystax, genus of Hucho, genus of Parahucho, genus of Salmo, genus of Salvethymus, etc. as a fish belonging to the order of Salmoniformes, but is not limited thereto.

In an example, the genus of Oncorhynchus may be one or more species selected from a group consisting of Chum salmon *(Oncorhynchus keta),* Coho salmon *(Oncorhynchus kisutch),* Sockeye salmon *(Oncorhynchus nerka),* Chinook salmon *(Oncorhynchus tshawytscha),* Pink salmon *(Oncorhynchus gorbuscha),* Trout *(Oncorhynchus masou),* Rainbow trout *(Oncorhynchus mykiss),* Cutthroat trout *(Oncorhynchus clarkii),* Golden trout *(Oncorhynchus aguabonita),* Apache trout *(Oncorhynchus apache),* Mexican golden trout *(Oncorhynchus chrysogaster),* Gila trout *(Oncorhynchus gilae),* Iwame trout *(Oncorhynchus iwame),* Black kokanee *(Oncorhynchus kawamurae),* etc. but is not limited thereto.

Fish of the genus of Salmo may be at least one species selected from a group consisting of Atlantic salmon *(Salmo salar),* Brown trout *(Salmo trutta),* etc., but is not limited thereto.

The feed composition may be rich in nutrients such as protein, fat, amino acid, fatty acid, minerals comprising phosphorus, iron, copper, manganese, zinc, etc. The nutrients are as described above.

The feed composition may comprise the biomass derived from microalgae in a content of 1 to 100 wt.%, 1 to 95 wt.%, 1 to 90 wt.%, 1 to 85 wt.%, 1 to 80 wt.%, 1 to 75 wt.%, 1 to 70 wt.%, 1 to 65 wt.%, 1 to 60 wt.%, 1 to 55 wt.%, 1 to 50 wt.%, 1 to 45 wt.%, 1 to 40 wt.%, 1 to 35 wt.%, 1 to 30 wt.%, 5 to 100 wt.%, 5 to 95 wt.%, 5 to 90 wt.%, 5 to 85 wt.%, 5 to 80 wt.%, 5 to 75 wt.%, 5 to 70 wt.%, 5 to 65 wt.%, 5 to 60 wt.%, 5 to 55 wt.%, 5 to 50 wt.%, 5 to 45 wt.%, 5 to 40 wt.%, 5 to 35 wt.%, 5 to 30 wt.%, 10 to 100 wt.%, 10 to 95 wt.%, 10 to 90 wt.%, 10 to 85 wt.%, 10 to 80 wt.%, 10 to 75 wt.%, 10 to 70 wt.%, 10 to 65 wt.%, 10 to 60 wt.%, 10 to 55 wt.%, 10 to 50 wt.%, 10 to 45 wt.%, 10 to 40 wt.%, 10 to 35 wt.%, 10 to 30 wt.%, 15 to 100 wt.%, 15 to 95 wt.%, 15 to 90 wt.%, 15 to 85 wt.%, 15 to 80 wt.%, 15 to 75 wt.%, 15 to 70 wt.%, 15 to 65 wt.%, 15 to 60 wt.%, 15 to 55 wt.%, 15 to 50 wt.%, 15 to 45 wt.%, 15 to 40 wt.%, 15 to 35 wt.%, 15 to 30 wt.%, 20 to 100 wt.%, 20 to 95 wt.%, 20 to 90 wt.%, 20 to 85 wt.%, 20 to 80 wt.%, 20 to 75 wt.%, 20 to 70 wt.%, 20 to 65 wt.%, 20 to 60 wt.%, 20 to 55 wt.%, 20 to 50 wt.%, 20 to 45 wt.%, 20 to 40 wt.%, 20 to 35 wt.%, 20 to 30 wt.%, 25 to 100 wt.%, 25 to 95 wt.%, 25 to 90 wt.%, 25 to 85 wt.%, 25 to 80 wt.%, 25 to 75 wt.%, 25 to 70 wt.%, 25 to 65 wt.%, 25 to 60 wt.%, 25 to 55 wt.%, 25 to 50 wt.%, 25 to 45 wt.%, 25 to 40 wt.%, 25 to 35 wt.%, 25 to 30 wt.%, 30 to 100 wt.%, 30 to 95 wt.%, 30 to 90 wt.%, 30 to 85 wt.%, 30 to 80 wt.%, 30 to 75 wt.%, 30 to 70 wt.%, 30 to 65 wt.%, 30 to 60 wt.%, 30 to 55 wt.%, 30 to 50 wt.%, 30 to 45 wt.%, 30 to 40 wt.%, or 30 to 35 wt.%, but is not limited thereto.

When the feed composition is fed to the fish of family Salmonidae, it may have excellent digestibility of the nutrients in the fish of family Salmonidae, such as true digestibility, apparent digestibility, etc.

The feed composition may comprise an indicator commonly used to measure the apparent digestibility (e.g., Chromium (III) oxide Cr₂O₃, Yttrium oxide (Y₂O₃), Ytterbium (III) oxide (Yb₂O₃), Lanthanum oxide (La₂O₃), Dysprosium (III) oxide (Dy₂O₃), etc.).

The apparent digestibility may refer to apparent digestibility of protein, apparent digestibility of lipid, apparent digestibility of energy, apparent digestibility of a micronutrient (e.g., apparent digestibility of phosphorus, manganese, zinc, copper, iron, selenium, etc.), apparent digestibility of fatty acid, etc.

The apparent digestibility of protein of the feed composition may be 80 to 100%, 80 to 98%, 80 to 96%, 80 to 95%, 80 to 92%, 80 to 90%, 80 to 88%, 82 to 100%, 82 to 98%, 82 to 96%, 82 to 95%, 82 to 92%, 82 to 90%, 82 to 88%, 84 to 100%, 84 to 98%, 84 to 96%, 84 to 95%, 84 to 92%, 84 to 90%, or 84 to 88%, but is not limited thereto.

The apparent digestibility of lipid of the feed composition may be 85 to 100%, 85 to 98%, 85 to 96%, 85 to 95%, 85 to 93%, 87 to 100%, 87 to 98%, 87 to 96%, 87 to 95%, 87 to 93%, 90 to 100%, 90 to 98%, 90 to 96%, 90 to 95%, 90 to 93%, 91 to 100%, 91 to 98%, 91 to 96%, 91 to 95%, or 91 to 93%, but is not limited thereto.

The apparent digestibility of ash of the feed composition may be 20 to 40%, 20 to 38%, 20 to 35%, 20 to 32%, 20 to 30%, 22 to 40%, 22 to 38%, 22 to 35%, 22 to 32%, 22 to 30%, 25 to 40%, 25 to 38%, 25 to 35%, 25 to 32%, 25 to 30%, 26 to 40%, 26 to 38%, 26 to 35%, 26 to 32%, 26 to 30%, 27 to 40%, 27 to 38%, 27 to 35%, 27 to 32%, 27 to 30%, 28 to 40%, 28 to 38%, 28 to 35%, 28 to 32%, or 28 to 30%, but is not limited thereto.

The digestibility of energy of the feed composition may be 80 to 100%, 80 to 98%, 80 to 96%, 80 to 95%, 80 to 92%, 80 to 90%, 80 to 88%, 82 to 100%, 82 to 98%, 82 to 96%, 82 to 95%, 82 to 92%, 82 to 90%, 82 to 88%, 84 to 100%, 84 to 98%, 84 to 96%, 84 to 95%, 84 to 92%, 84 to 90%, or 84 to 88%, but is not limited thereto.

The apparent digestibility of ash (sum of inorganic substances) which is a micronutrient, or each inorganic substance (e.g., calcium, phosphorus, magnesium, sodium, potassium, chlorine, sulfur, iron, iodine, zinc, copper, selenium, manganese, chromium, cobalt, molybdenum, fluorine, etc.) of the feed composition may be high.

The apparent digestibility of phosphorus of the feed composition may be 30 to 70%, 30 to 67%, 30 to 65%, 30 to 62%, 30 to 60%, 30 to 57%, 30 to 55%, 30 to 52%, 30 to 50%, 32 to 70%, 32 to 67%, 32 to 65%, 32 to 62%, 32 to 60%, 32 to 57%, 32 to 55%, 32 to 52%, 32 to 50%, 35 to 70%, 35 to 67%, 35 to 65%, 35 to 62%, 35 to 60%, 35 to 57%, 35 to 55%, 35 to 52%, 35 to 50%, 37 to 70%, 37 to 67%, 37 to 65%, 37 to 62%, 37 to 60%, 37 to 57%, 37 to 55%, 37 to 52%, 37 to 50%, 40 to 70%, 40 to 67%, 40 to 65%, 40 to 62%, 40 to 60%, 40 to 57%, 40 to 55%, 40 to 52%, 40 to 50%, 42 to 70%, 42 to 67%, 42 to 65%, 42 to 62%, 42 to 60%, 42 to 57%, 42 to 55%, 42 to 52%, 42 to 50%, 45 to 70%, 45 to 67%, 45 to 65%, 45 to 62%, 45 to 60%, 45 to 57%, 45 to 55%, 45 to 52%, 45 to 50%, 47 to 70%, 47 to 67%, 47 to 65%, 47 to 62%, 47 to 60%, 47 to 57%, 47 to 55%, 47 to 52%, or 47 to 50%, but is not limited thereto.

The apparent digestibility of iron of the feed composition may be 1 mg/kg or more, 2 mg/kg or more, 3 mg/kg or more, 4 mg/kg or more, 5 mg/kg or more, 6 mg/kg or more, 7 mg/kg or more, 8 mg/kg or more, 9 mg/kg or more, 10 mg/kg or more, 1 to 20 mg/kg, 1 to 18 mg/kg, 1 to 15 mg/kg, 1 to 12 mg/kg, 1 to 11 mg/kg, 2 to 20 mg/kg, 2 to 18 mg/kg, 2 to 15 mg/kg, 2 to 12 mg/kg, 2 to 11 mg/kg, 5 to 20 mg/kg, 5 to 18 mg/kg, 5 to 15 mg/kg, 5 to 12 mg/kg, 5 to 11 mg/kg, 8 to 20 mg/kg, 8 to 18 mg/kg, 8 to 15 mg/kg, 8 to 12 mg/kg, 8 to 11 mg/kg, 10 to 20 mg/kg, 10 to 18 mg/kg, 10 to 15 mg/kg, 10 to 12 mg/kg, or 10 to 11 mg/kg, but is not limited thereto.

The apparent digestibility of copper of the feed composition may be 1 mg/kg or more, 5 mg/kg or more, 10 mg/kg or more, 11 mg/kg or more, 12 mg/kg or more, 13 mg/kg or more, 14 mg/kg or more, 15 mg/kg or more, 16 mg/kg or more, 17 mg/kg or more, 1 to 30 mg/kg, 1 to 28 mg/kg, 1 to 25 mg/kg, 1 to 22 mg/kg, 1 to 20 mg/kg, 1 to 18 mg/kg, 2 to 30 mg/kg, 2 to 28 mg/kg, 2 to 25 mg/kg, 2 to 22 mg/kg, 2 to 20 mg/kg, 2 to 18 mg/kg, 5 to 30 mg/kg, 5 to 28 mg/kg, 5 to 25 mg/kg, 5 to 22 mg/kg, 5 to 20 mg/kg, 5 to 18 mg/kg, 8 to 30 mg/kg, 8 to 28 mg/kg, 8 to 25 mg/kg, 8 to 22 mg/kg, 8 to 20 mg/kg, 8 to 18 mg/kg, 10 to 30 mg/kg, 10 to 28 mg/kg, 10 to 25 mg/kg, 10 to 22 mg/kg, 10 to 20 mg/kg, 10 to 18 mg/kg, 14 to 30 mg/kg, 14 to 28 mg/kg, 14 to 25 mg/kg, 14 to 22 mg/kg, 14 to 20 mg/kg, 14 to 18 mg/kg, 15 to 30 mg/kg, 15 to 28 mg/kg, 15 to 25 mg/kg, 15 to 22 mg/kg, 15 to 20 mg/kg, or 15 to 18 mg/kg, but is not limited thereto.

The apparent digestibility of manganese of the feed composition may be 1 mg/kg or more, 2 mg/kg or more, 3 mg/kg or more, 4 mg/kg or more, 5 mg/kg or more, 6 mg/kg or more, 7 mg/kg or more, 8 mg/kg or more, 9 mg/kg or more, 10 mg/kg or more, 1 to 20 mg/kg, 1 to 18 mg/kg, 1 to 15 mg/kg, 1 to 12 mg/kg, 1 to 11 mg/kg, 2 to 20 mg/kg, 2 to 18 mg/kg, 2 to 15 mg/kg, 2 to 12 mg/kg, 2 to 11 mg/kg, 5 to 20 mg/kg, 5 to 18 mg/kg, 5 to 15 mg/kg, 5 to 12 mg/kg, 5 to 11 mg/kg, 8 to 20 mg/kg, 8 to 18 mg/kg, 8 to 15 mg/kg, 8 to 12 mg/kg, 8 to 11 mg/kg, 10 to 20 mg/kg, 10 to 18 mg/kg, 10 to 15 mg/kg, 10 to 12 mg/kg, or 10 to 11 mg/kg, but is not limited thereto.

The apparent digestibility of zinc of the feed composition may be 1 mg/kg or more, 5 mg/kg or more, 10 mg/kg or more, 15 mg/kg or more, 20 mg/kg or more, 25 mg/kg or more, 30 mg/kg or more, 1 to 50 mg/kg, 1 to 45 mg/kg, 1 to 40 mg/kg, 1 to 35 mg/kg, 5 to 50 mg/kg, 5 to 45 mg/kg, 5 to 40 mg/kg, 5 to 35 mg/kg, 10 to 50 mg/kg, 10 to 45 mg/kg, 10 to 40 mg/kg, 10 to 35 mg/kg, 15 to 50 mg/kg, 15 to 45 mg/kg, 15 to 40 mg/kg, 15 to 35 mg/kg, 20 to 50 mg/kg, 20 to 45 mg/kg, 20 to 40 mg/kg, 20 to 35 mg/kg, 25 to 50 mg/kg, 25 to 45 mg/kg, 25 to 40 mg/kg, 25 to 35 mg/kg, 27 to 50 mg/kg, 27 to 45 mg/kg, 27 to 40 mg/kg, or 27 to 35 mg/kg, but is not limited thereto.

The biomass derived from microalgae or the feed composition comprising the biomass derived from microalgae has excellent digestibility of the nutrients and may be usefully used as the feed for the fish of family Salmonidae. The biomass derived from microalgae or the feed composition comprising the biomass derived from microalgae may be used for improving (increasing) the digestibility of the fish of family Salmonidae.

Another aspect provides a method of manufacturing a feed composition for fish of family Salmonidae comprising the biomass derived from microalgae. The method may comprise manufacturing a feed mixture by mixing a feed and a binder after adding water to the feed mixture and mixing, and then forming the mixture into a predetermined shape.

The binder may be added to improve the binding power and water absorption power of the feed and may comprise at least one carbohydrate component selected from a group consisting of a grain powder selected from wheat flour, corn flour, rice flour, barley flour, potato flour, and sweet potato flour, starch selected from tapioca starch, potato starch, sweet potato starch, rice starch, oat starch, soybean starch, barley starch, and modified starch thereof. The modified starch refers to natural starch that has been chemically, physically or enzymatically treated and may be, for example, dextrin, oxidized starch, oxygenated starch, alpha (α) starch, starch derivative, etc., but is not limited thereto.

The molding of the feed mixture may be performed using a conventionally known instrument and preferably may be performed using any one selected from an extruder, an impeller-type mixer, and a kneader. Through this molding process, the feed mixture may be molded into granule, pill, or pellet shape.

The method of manufacturing the feed composition may further comprise drying the feed mixture as needed after the molding the feed mixture is completed. In this case, as the drying method, any conventionally known drying method may be used without limitation, and for example, a natural drying, a hot air drying, or a high-temperature drying method may be used. In addition, during a drying process, the feed for fish aquaculture may be dried to a semi-dry state comprising some moisture or may be dried to a completely dry state.

Still another aspect provides a method of aquaculture for fish of family Salmonidae aquaculture feeding a feed composition for the fish of family Salmonidae comprising biomass derived from microalgae to the fish of family Salmonidae, or a method of improving (increasing) digestibility of the fish of family Salmonidae, or a use for using in improving (increasing) the digestibility of the fish of family Salmonidae of the biomass derived from microalgae or the feed composition for the fish of family Salmonidae comprising the biomass derived from microalgae.

The feed composition and the fish of family Salmonidae are as described above.

By feeding the feed composition comprising the biomass derived from microalgae to the fish of family Salmonidae, the digestibility of the fish of family Salmonidae increases, thereby efficient aquaculturing the fish of family Salmonidae.

The digestibility of the fish of family Salmonidae may be nutrient digestibility of the fish of family Salmonidae and may be true digestibility, apparent digestibility, etc.

The apparent digestibility of the fish of family Salmonidae may refer to the apparent digestibility of protein, the apparent digestibility of lipid, the apparent digestibility of energy, the apparent digestibility of micronutrient (e.g., the apparent digestibility of phosphorus, manganese, zinc, copper, iron, selenium, etc.), the apparent digestibility of fatty acid or ash (sum of inorganic substances) which is a micronutrient, or the apparent digestibility of each inorganic substance (e.g., calcium, phosphorus, magnesium, sodium, potassium, chlorine, sulfur, iron, iodine, zinc, copper, selenium, manganese, chromium, cobalt, molybdenum, fluorine, etc.).

The digestibility of the feed composition comprising the biomass derived from microalgae may have increased by 1% or more, 2% or more, 3% or more, 4% or more, 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 100% or more, 105% or more, or 110% or more compared to a control group (e.g., a feed composition not comprising the biomass derived from microalgae).

In an example, the digestibility of phosphorus of the feed composition comprising the biomass derived from microalgae may have increased by 5% or more, 10% or more, 15% or more, or 20% or more compared to the control group. In an example, the digestibility of iron of the feed composition comprising the biomass derived from microalgae may have increased by 10% or more, 20% or more, 30% or more, 40% or more, 50% or more, 60% or more, 70% or more, 80% or more, 90% or more, or 100% or more compared to the control group. In an example, the digestibility of copper of the feed composition comprising the biomass derived from microalgae may have increased by 5% or more or 10% or more compared to the control group. In an example, the digestibility of manganese of the feed composition comprising biomass derived from microalgae may have increased by 10% or more, 20% or more, 30% or more, or 40% or more compared to the control group. In an example, the digestibility of zinc of the feed composition comprising the biomass derived from microalgae may have increased by 5% or more, 10% or more, or 15% or more compared to the control group.

### ADVANTAGEOUS EFFECTS

A feed composition comprising a biomass derived from microalgae of the present application can be usefully used as a feed composition for fish by using the microalgae having high protein, high fat, and high mineral content without any factors that deteriorate the feed quality.

### BRIEF DESCRIPTION OF THE DRAWINGS

FIG. 1 is a graph comparing apparent digestibility of trace elements (minerals: phosphorus, iron, copper, manganese, zinc) of a test feed (Algal Protein diet) with that of a comparative feed (SPC diet) and a standard feed (Reference diet).
FIG. 2 is a graph comparing the apparent digestibility of dietary fatty acids (total saturated fatty acid, total monounsaturated fatty acid, total omega-6 fatty acid, total omega-3 fatty acid, total polyunsaturated fatty acid, EPA+DHA) of the test feed (Algal protein diet) with that of the standard feed (Reference diet).

### MODE FOR INVENTION

Hereinafter, the present application will be described in more detail with the following Examples. However, Examples below are provided to exemplify the present application, and the scope of the present application is not limited by Examples below and would be apparent to those skilled in the art to which the present application pertains.

Accordingly, the present invention will be described in more detail by Examples.

### Example 1: Manufacturing test feed (Test diet)

In order to produce an efficient feed, it is necessary to evaluate the quality compared to the price of a feed raw material. Among methods of evaluating the feed raw material, measuring digestibility of nutrient of the raw material is one of the most basic methods, and among the methods of measuring the digestibility of nutrient of fish, the digestibility was measured indirectly by mixing an indicator substance such as yttrium oxide with the feed. In addition, the evaluation of the digestibility of nutrient of the feed raw material is generally conducted by supplying a test feed which is manufactured by mixing a basic feed (standard feed) and the feed raw material to be evaluated in a ratio of 7:3 to fish. This is to minimize an influence of other feed raw material on the feed raw material to be evaluated.

### 1-1 Nutrient and component analysis by experimental components

In the present Example, a biomass derived from microalgae (Biomass derived from Algae) as the feed raw material to be tested, fish meal/fish oil as a raw material for a standard feed, and soy protein concentrate (SPC, CJ CheilJedang product) which is a plant-based alternative protein as the raw material for a comparative feed were selected. The biomass derived from microalgae was manufactured using a microalgae of genus Schizochytrium (Schizochytrium sp., KCTC14345BP). Specifically, the biomass derived from microalgae was manufactured through a series of processes of mass culturing, drying, and packaging the microalgae of the genus Schizochytrium, a culture solution of the microalgae of the genus Schizochytrium cultured at a high concentration through the culturing process was transferred to a purification process, moisture content in the culture solution was reduced through a dehydration process, and it was dried and powdered using a drum drying or spray drying method. A finally obtained powder of the microalgae of the genus Schizochytrium was made commercially available by packaging in a preservation process and was used in following Examples.

For using as a salmon feed, content of major nutrients such as a crude protein, fat, ash, and moisture, and trace nutrients (minerals) such as a fatty acid, phosphorus, manganese, and zinc of each raw material were analyzed. A nutritional composition by the raw material is as shown in Table 2 below.

**[Table 2]**

| Analysis | | Unit | Microalgae protein | Soy protein concentrat e (SPC) | Fish meal | | |
|---|---|---|---|---|---|---|---|
| Crude protein | | wt.% | 73.7 | 64.2 | 66.4 | | |
| (Kieldahl) | | | | | | | |
| Ash | wt.% | 7.9 | | 6.6 | | 13.2 | |
| Moisture | wt.% | 3.3 | | 4.6 | | 7 | |
| Fat (Bligh & Dyer) | wt.% | 13.8 | | 1.2-1.5 | | 10.78 | |
| Total P | wt.% | 0.64 | | 0.72-0.78 | | 1.7 | |
| Total amino acids | | | | | | | |
| Alanine | g/100g | 2.2 | | 2.8 | | 4.3 | |
| Arginine | g/100g | 8.2 | | 4.4 | | 4.3 | |
| Aspartic acid | g/100g | 3.3 | | 7.3 | | 6.3 | |
| Phenylalanine | g/100g | 1.5 | | 3.2 | | 2.6 | |
| Glutamine | g/100g | >20.0 | | 11.6 | | 9.2 | |
| Glycine | g/100g | 1.8 | | 2.7 | | 4.3 | |
| Histidine | g/100g | 0.76 | | 1.6 | | 1.6 | |
| Hydroxyproline | g/100g | <0.10 | | 0.18 | | 0.58 | |
| Isoleucine | g/100g | 1.5 | | 2.9 | | 3 | |
| Leucine | g/100g | 2.4 | | 4.8 | | 5.3 | |
| Lysine | g/100g | 2.4 | | 4.4 | | 5.4 | |
| Methionine | g/100g | 0.89 | | 0.82 | | 2.2 | |
| Proline | g/100g | 3.4 | | 3.1 | | 3 | |
| Serine | g/100g | 1.8 | | 3.3 | | 2.8 | |
| Threonine | g/100g | 1.6 | | 2.4 | | 2.9 | |
| Tyrosine | g/100g | 1.2 | | 2.2 | | 2.1 | |
| Valine | g/100g | 2 | | 3 | | 3.4 | |
| Tryptophan | g/100g | 0.53 | | 0.77 | | | |
| Cysteine | g/100g | | | 0.9 | | | |

| Fatty acids | Unit (wt.%) | Microalga e protein | Soy protein concentrate | | Fish meal | | Fish oil |
|---|---|---|---|---|---|---|---|
| Total saturated fatty acids | g/100g lipid | 26.4 | 0.39 | | 19.7 | | 20.6 |
| Total monounsaturated fatty acids | g/100g **lipid** | 0.9 | 0.4 | | 31.4 | | 50.5 |
| Total (n-6) fatty acids | g/100g lipid | 0.5 | 0.91 | | 2.9 | | 1.9 |
| Total (n-3) fatty acids | g/100g lipid | 35.2 | 0.1 | | 22.7 | | 18.5 |
| EPA + DHA | g/100g lipid | 34 | - | | 18.5 | | 13.5 |
| Total polyunsaturated fatty acids | g/100g lipid | 35.7 | 1.01 | | 26.1 | | 21 |

In the Table 2, the composition (wt.%) of the crude protein, a crude fiber, ash, moisture, and fat of each raw material (biomass derived from microalgae, concentrated soy protein concentrate, fish meal) was analyzed, and remaining ingredients comprise nitrogen free extracts such as starch, sugar, cellulose and lignin. Phosphorus that is comprised in the ash is a separately analyzed item that is comprised in the ash. In the Table 2, a content of the amino acid refers to weight of each amino acid measured based on 100 g of the total amino acid. In the Table 2, a content of the fatty acid refers to weight of each fatty acid measured based on 100 g of the total lipid. In the fatty acid content of the soy protein concentrate in the Table 2, a total content of the saturated fatty acid corresponds to Palmitic acid (C16:0) 0.31 and stearic acid (C18:0) 0.08, a total content of the monounsaturated fatty acid corresponds to oleic acid (C18:1 n-9) 0.4, a total content of the omega-6 fatty acid corresponds to Linoleic acid (C18:2 n-6) 0.91, and a total content of the omega-3 fatty acid corresponds to alpha-Linolenic acid (C18:3 n-3) 0.1.

### 1-2 Feed manufacturing and composition

The standard feed was manufactured from a feed mixture comprising fish meal, fish oil, wheat, vitamin, and mineral mixture.

**[Table 3]**

| | Standard feed (wt.%) | 70 wt.% of standard feed (For manufacturing test feed and comparative feed) |
|---|---|---|
| Fish meal | 68.540 | 47.978 |
| Fish oil | 15.00 | 10.50 |
| Wheat | 15.00 | 10.50 |
| Vitamin (vitamins mix) | 0.720 | 0.504 |
| Mineral (minerals mix) | 0.720 | 0.504 |
| Yttrium oxide | 0.020 | 0.014 |

The standard feed was manufactured with the composition shown in the Table 3, and the test feed and the comparative feed were manufactured by replacing 30 wt.% of the standard feed with the biomass derived from microalgae or the soy protein concentrate, respectively. A nutritional composition table of the test feed, the comparative feed, and the standard feed is shown in Table 4 below.

**[Table 4]**

| Analysis | Unit | Test feed | Comparative feed | Standard feed |
|---|---|---|---|---|
| Crude protein (Kjeldahl) | wt.% | 57 | 51.6 | 49.5 |
| Ash | wt.% | 9.5 | 8.9 | 10.1 |
| Moisture | wt.% | 7.2 | 10.2 | 9.3 |
| Fat (B&D) | wt.% | 20.1 | 17.1 | 22.1 |
| Crude energy | kJ/g | 21.25 | 20.26 | 21.4 |
| Phosphorus(P) | mg/kg | 14000 | 12000 | 15000 |
| Selenium(Se) | mg/kg | 1.4 | 2.5 | 2.3 |
| Iron(Fe) | mg/kg | 120 | 150 | 160 |
| Copper(Cu) | mg/kg | 13 | 12 | 13 |
| Manganese(Mn) | mg/kg | 38 | 48 | 50 |
| Zinc(Zn) | mg/kg | 160 | 170 | 220 |
| Total amino acids | | | | |
| Hydroxyproline | g/100g | 0 | 0.3 | 0.4 |
| Alanine | g/100g | 2.2 | 2.9 | 3.1 |
| Arginine | g/100g | 8.2 | 3.4 | 3 |
| Aspartic acid | g/100g | 3.3 | 5.1 | 4.4 |
| Phenylalanine | g/100g | 1.5 | 2.2 | 1.9 |
| Glutamine | g/100g | >20.0 | 7.9 | 6.8 |
| Glycine | g/100g | 1.8 | 2.6 | 2.7 |
| Histidine | g/100g | 0.8 | 1.3 | 1.2 |
| Isoleucine | g/100g | 1.5 | 2.2 | 2 |
| Leucine | g/100g | 2.4 | 3.7 | 3.5 |
| Lysine | g/100g | 2.4 | 3.7 | 3.9 |
| Methionine | g/100g | 0.9 | 1.1 | 1.4 |
| Proline | g/100g | 3.4 | 2.3 | 2.1 |
| Serine | g/100g | 1.8 | 2.3 | 2.1 |
| Threonine | g/100g | 1.6 | 2.1 | 2.1 |
| Tyrosine | g/100g | 1.2 | 1.6 | 1.6 |
| Valine | g/100g | 2 | 2.4 | 2.4 |
| Fatty acids | | | | |
| Total saturated fatty acids | g/100g lipid | 3.64 | 0.05 | 2.03 |
| Total monounsaturated fatty acids | g/100g lipid | 0.12 | 0 | 3.11 |
| Total (n-6) fatty acids | g/100g lipid | 0.07 | 0.126 | 0.49 |
| Total (n-3) fatty acids | g/100g lipid | 4.86 | 0.01 | 2.06 |
| Total polyunsaturated fatty acids | g/100g lipid | 4.93 | - | 2.6 |
| EPA + DHA | g/100g lipid | 4.69 | 0.13 | 1.67 |

In the Table 4, the composition (wt.%) of the crude protein, ash, moisture, and fat of each feed (test feed, comparative feed, standard feed) was analyzed, and remaining ingredients comprise nitrogen free extracts such as starch, sugar, cellulose and lignin, and crude fiber, etc. Minerals such as phosphorus, selenium, iron, copper, manganese, and zinc were measured in mg/kg compared to 1 kg of the total feed. In the Table 4, a content of the amino acid refers to weight of each amino acid measured based on 100 g of the total amino acid. In the Table 4, a content of the fatty acid refers to weight of each fatty acid measured based on 100 g of the total lipid.

In order to evaluate a definite digestibility efficacy of the nutrient in Atlantic salmon fry (Atlantic salmon smolt, obtained from Nofima, Norway) when manufacturing the test feed and the comparative feed, 70 wt.% of the standard feed was replaced by 30 wt.% of each raw material (biomass derived from microalgae and SPC) using a standard partial substitution method, and then Yttrium oxide (Y₂O₃, obtained from Sigma Aldrich, product number 205168) which is a digestibility indicator was added for analysis.

### 1-3 Analysis of experimental feed and ingredient of fish body

The raw material, feed, and feces were analyzed for crude protein (Kjeldahl method Nx 6.25; ISO 5983-1997), moisture (ISO 6496-1999), ash (ISO 5984-2002), and lipid to confirm an approximate composition thereof. A fatty acid profile was experimented using a micro-GC gas chromatograph according to the AOCS official method Ce 1b-89. Total and soluble phosphorus were measured using a spectrophotometer (ISO 6491-1998).

### Example 2: Experimental fish and tank setup

The Atlantic salmon fry (Nofima, Norway) used in the experiment had an average weight of 105 g, 100 salmons were accommodated in each tank and randomly placed in three repetitions per experimental group, and water temperature was always maintained at 12 °C. A breeding experiment was conducted for 4 weeks. During the experimental period, oxygen saturation was measured three times in all test tanks for water quality management and was found to be similar in all test tanks.

A pre-weighed amount of each experimental feed was supplied to the experimental tanks before introduction of the experimental fish group. Each feed (test feed, comparative feed, standard feed) was supplied to the tank in a pellet form, and the pellets were recovered by a sieve for a certain period of time corresponding to a time between the feed supply and feed collection during the experiment and washed with discharged water. Thereafter, the feed pellets were weighed again to measure dry matter solubility (moisture stability) of each feed. This value was used to monitor a daily feed intake of each experimental group by recalculating a total content of unconsumed feed per day for each tank.

That is, in order to estimate the total daily feed intake of the experimental fish group, the unconsumed feed was collected and weighed daily.

### Example 3: Sampling and digestibility calculation

After the experiment was completed, all fish in each tank were removed, and after collecting the feces in one box for each tank, the recovered feces were freeze-dried and pulverized, and then stored at -20 °C before analysis. An average weight was calculated by applying a bulk-weight to weight of the fish in each tank.

A content of yttrium oxide (Y₂O₂) (digestibility indicator) in the experimental feed and the feces was analyzed using ICP-OES (Agilent 5110 VDV, inductively coupled plasma optical emission spectrometer) equipment (reference: NS:EN 15641:2017).

### Example 4: Comparison of digestibility for each feed

### 4-1 Digestibility of nutrient

The apparent digestibility of the nutrient (protein, lipid, ash, energy) of the test feed, the comparative feed, and the standard feed for the Atlantic salmon fry is shown in Table 5 below.

**[Table 5]**

| Apparent digestibility (Unit: %) | Test feed | Comparative feed | Standard feed |
|---|---|---|---|
| ADC protein | 85.03±0.28 | 85.96±0.20 | 86.35±0.18 |
| ADC lipids | 92.93±0.12 | 94.92±0.10 | 95.48±0.20 |
| Ash (%) | 28.61±2.50 | 25.80±0.96 | 31.66±2.32 |
| ADC energy | 85.12±0.53 | 82.57±0.16 | 87.53±0.21 |

As can be confirmed in the Table 5, the test feed showed a similar level of digestibility of protein and digestibility of lipid compared to the comparative feed and the standard feed. In particular, the test feed was confirmed to have higher digestibility of ash and digestibility of energy compared to those of the comparative feed. This result indicates that the test feed comprising the biomass derived from microalgae of the present application may be usefully used as the fish feed.

### 4-2 Digestibility of micronutrient

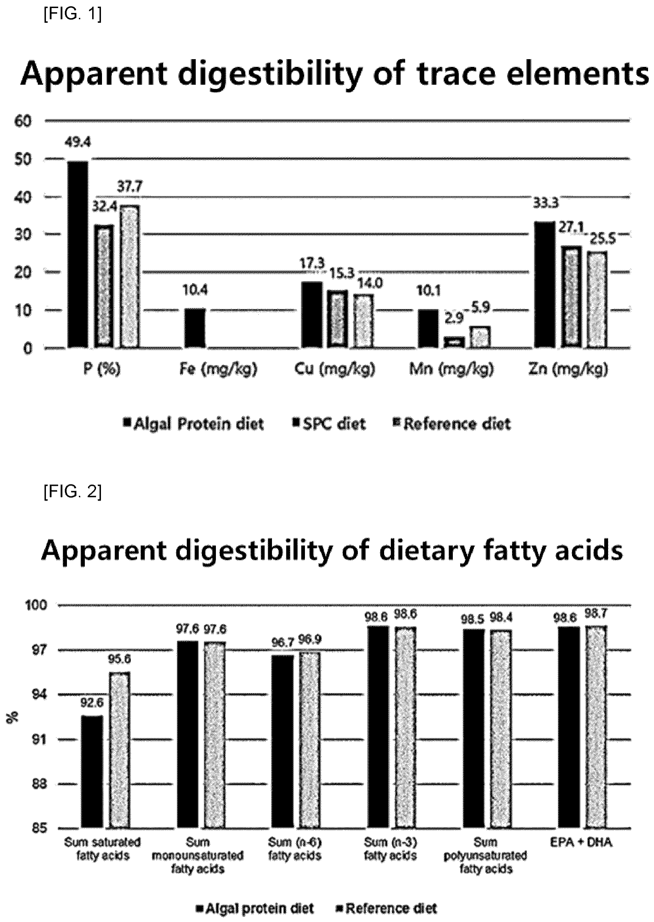

The apparent digestibility of the micronutrient (phosphorus, iron, copper, manganese, zinc) of the test feed, the comparative feed, and the standard feed for the Atlantic salmon fry is shown in Table 5 below and FIG. 1.

Fish, like a terrestrial animal, requires various minerals as a nutrient source comprising phosphorus (P), manganese (Mn), zinc (Zn), copper (Cu), iron (Fe), and selenium (Se). In terms of their respective functions and roles, phosphorus (P) is effective in skeletal tissue and phospholipid, copper (Cu) and zinc (Zn) are effective as metalloenzymes, iron (Fe) is effective for hemoglobin, manganese (Mn) is effective in skeletal tissue and enzyme reaction, and selenium (Se) has an antioxidant property.

As can be confirmed in Table 6 below and FIG. 1, the test feed had higher digestibility of ash and phosphorus compared to that of the comparative feed, and in particular, the digestibility of the phosphorus in the test feed was higher than that of the standard feed.

This is because all phosphorus (P) in the biomass derived from microalgae raw material is water-soluble, but the phosphorus (P) in fish meal is partially bound to hydroxyapatite derived from a bone which is difficult to digest.

In addition, although the biomass derived from microalgae raw material comprises relatively high levels of phospholipids, the phosphorus (P) in a plant-derived protein (e.g., plant bran, seed, etc.) exists mostly in a form of inositol phosphate (e.g., phytic acid, phytate) having low availability, which is known to inhibit intestinal absorption of other dietary minerals (e.g., Ca, Fe, Zn). In addition to the phosphorus (P), the test feed showed significantly superior digestibility of iron (Fe), copper (Cu), manganese (Mn), and zinc (Zn) which are essential trace elements digestible by salmon compared to those of the comparative feed and the standard feed.

**[Table 6]**

| Table 6. Apparent digestibility of the micronutrient contained in the experimental feed | | | |
|---|---|---|---|
| | Test feed | Comparative feed | Standard feed |
| Ash(%) | 28.61±2.50 | 25.80±0.96 | 31.66±2.32 |
| Total P (%) | 49.41±2.73 | 32.41±0.98 | 37.73±1.71 |
| Fe (mg/kg) | 10.42±2.09 | -1.32±3.46 | -0.18±7.60 |
| Cu (mg/kg) | 17.31±1.93 | 15.28±3.90 | 14.02±3.45 |
| Mn (mg/kg) | 10.08±3.80 | 2.90±2.95 | 5.85±3.83 |
| Zn (mg/kg) | 33.34±1.81 | 27.07±0.12 | 25.47±0.58 |

### 4-3 Digestibility of fatty acid

The apparent digestibility of the fatty acid of the test feed and the standard feed for the Atlantic salmon fry is shown in Table 7 below and FIG. 2.

The fatty acid in the feed may directly affect the quality of the fish body in relation to the fatty acid composition of the fish body. As can be confirmed in Table 6 below, the test feed comprised an unsaturated fatty acid at an equal or higher level compared to that of the standard feed. That is, the test feed, similar to the comparative feed, contains a large amount of the unsaturated fatty acid such as an eicosapentaenoic acid (EPA (C20:5 n-3)) and docosahexaenoic acid (DHA (C22:6 n-3)) which are essential fatty acids, and thus the test feed may be used as a good raw material for supplying the essential fatty acid for fish. In addition, the saturated fatty acid has a melting point higher than that of the unsaturated fatty acid and may exist as a solid in water at room temperature or low temperature. Therefore, the test feed has a lower content of the saturated fatty acid compared to the standard feed and may be suitably used as the fish feed.

**[Table 7]**

| Fatty acid | | Test feed | Standard feed |
|---|---|---|---|
| Total saturated fatty acids | | 92.61±0.23 | 95.55±0.10 |
| Total monounsaturated fatty acids | | 97.61±0.16 | 97.58±0.09 |
| Total (n-6) fatty acids | | 96.67±0.26 | 96.91±0.32 |
| Total (n-3) fatty acids | | 98.63±0.05 | 98.59±0.14 |
| | C18:3 n-3 | 98.67±0.24 | 98.73±0.20 |
| | C18:4 n-3 | 99.66±0.30 | 99.44±0.15 |
| | C20:4 n-3 | 100.00±0.00 | 98.84±0.08 |
| | C20:5 n-3 (EPA) | 99.20±0.11 | 99.11±0.19 |
| | C21:5 n-3 | 100.00±0.00 | 100.00±0.00 |
| | C22:5 n-3 | 98.70±1.15 | 98.66±0.05 |
| | C22:6 n-3 (DHA) | 98.34±0.07 | 98.37±0.10 |
| Total polyunsaturated fatty acids | | 98.46±0.07 | 98.38±0.17 |
| EPA + DHA | | 98.56±0.07 | 98.67±0.14 |

### Example 5: Digestibility of feed raw material (Partial ADC of test ingredient nutrients)

In Example 5, the digestibility of nutrient of each raw material itself comprised in the feed (test feed, comparative feed, standard feed) of Example 4 was measured.

### 5-1 Digestibility of nutrient

The digestibility of nutrient of the raw material of the feed was measured and shown in Table 8 below.

**[Table 8]**

| Nutrient | Test feed | Comparative feed | Standard feed |
|---|---|---|---|
| Protein (%) | 82.9±0.7 | 85.2±0.6 | 86.3±0.2 |
| Fat (%) | 89.2±0.3 | - | 95.5±0.2 |
| Fe (%) | 10.4±2.1 | -1.3±3.5 | -0.2±7.6 |
| Cu (%) | 17.3±1.9 | 15.3±3.9 | 14.0±3.4 |
| Mn (%) | 10.1±3.8 | 2.9±2.9 | 5.8±3.8 |
| Zn (%) | 33.3±1.8 | 27.1±0.1 | 25.5±0.6 |
| Total P (%) | 75.7±8.9 | 19.3±3.4 | 37.7±1.7 |

As can be confirmed in the Table 8, the digestibility of protein and the digestibility of fat of the raw material of the test feed were similar to those of the comparative feed and standard feed, and the digestibility of iron, copper, manganese, zinc, and phosphorus was confirmed to be significantly superior compared to those of the comparative feed and standard feed.

### 5-2 Digestibility of fatty acid

The digestibility of fatty acid of the raw material of the feed was measured and shown in Table 9 below.

**[Table 9]**

| Fatty acid | | Test feed | Standard feed |
|---|---|---|---|
| Total saturated fatty acids | | 89.4±0.5 | 95.5±0.1 |
| | C14:0 | 90.4±0.7 | 96.9±0.1 |
| | C16:0 | 88.9±0.5 | 95.2±0.1 |
| | C18:0 | 82.3±0.4 | 93.6±0.2 |
| Total mono-unsaturated fatty acids | | 99.1±8.1 | 97.6±0.1 |
| | C18:1 (n-9)+(n-7) | 95.6±10.9 | 98.0±0.3 |
| | C22:1 (n-11)+(n-9)+(n-7) | 109.1±13.6 | 97.2±0.1 |
| | C24:1 n-9 | 84.2±0.7 | 92.4±0.6 |
| Total (n-6) fatty acids | | 93.2±4.0 | 96.9 ±0.3 |
| | C20:4 n-6 (ARA) | 107.0±0.0 | 97.6±0.1 |
| Sum (n-3) fatty acids | | 98.7±0.1 | 98.6±0.1 |
| | C18:4 n-3 | 102.0±3.4 | 99.4±0.1 |
| | C20:4 n-3 | 101.7±0.0 | 98.8 b±0.1 |
| | C20:5 n-3 (EPA) | 99.9±1.1 | 99.1±0.2 |
| | C22:5 n-3 | 98.8±3.7 | 98.7±0.1 |
| | C22:6 n-3 (DHA) | 98.3±0.1 | 98.4±0.1 |
| Sum polyunsaturated fatty acids | | 98.5±0.1 | 98.4±0.2 |
| | EPA + DHA | 98.5±0.1 | 98.7±0.1 |

As can be confirmed in the Table 9, the apparent digestibility of unsaturated fatty acid of the test feed was similar to or higher compared to that of the standard feed. In addition, the test feed is an essential fatty acid required by marine fish, and it was confirmed that the apparent digestibility of a highly unsaturated fatty acid (HUFA) of omega-3 series such as eicosapentaenoic acid (EPA) and docosahexaenoic acid (DHA) which are functional substances contained in large content in fish oil is at an equivalent level to that of the comparative feed. In addition, the apparent digestibility of saturated fatty acid of the test feed is lower than that of the standard feed, thereby achieving advantageous effects when used as the fish feed.

From the foregoing, those skilled the art to which the present invention pertains will be able to understand that the present invention may be embodied in other specific forms without modifying the technical concepts or essential characteristics of the present invention. In this regard, Examples disclosed herein are only for illustrative purposes and should not be construed as limiting the scope of the present invention. Furthermore, the scope of the present invention is defined by the appended claims rather than the detailed description, and it should be understood that all modifications or variations derived from the meanings and scope of the present invention and equivalents thereof are comprised in the scope of the appended claims.

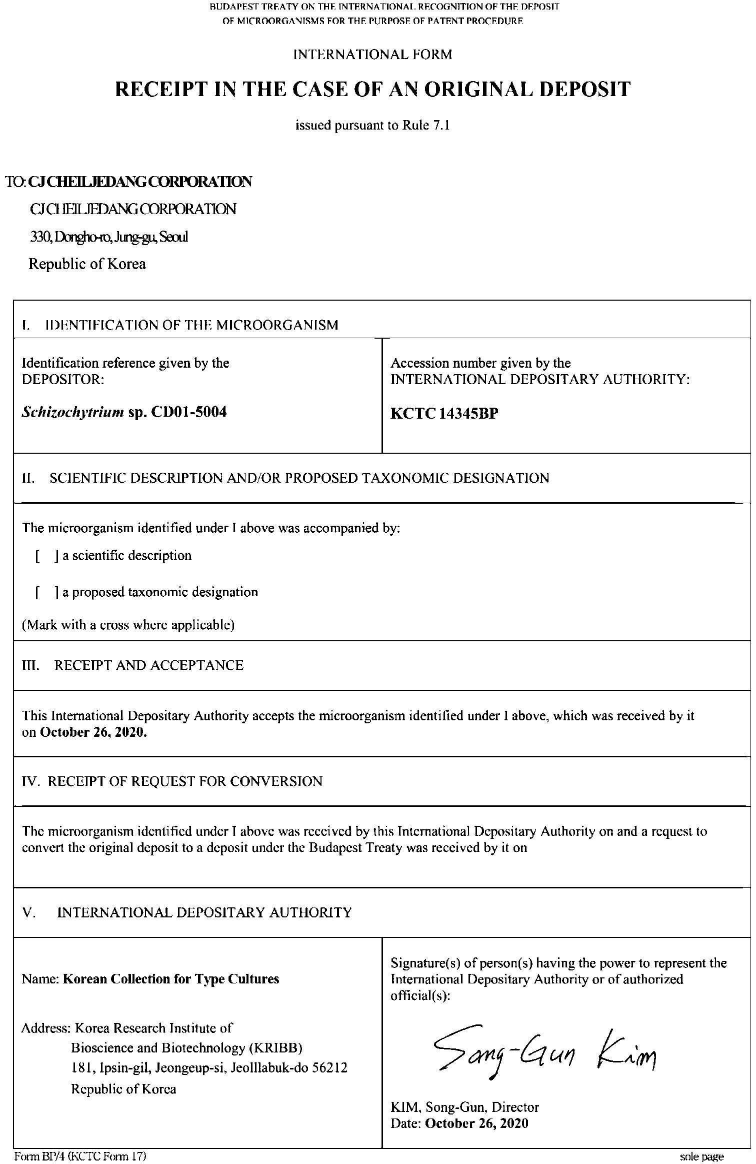

## Claims

1. A feed composition for fish of family Salmonidae, comprising a biomass derived from microalgae.

2. The feed composition of claim 1, wherein the microalgae is a microalgae of genus Schizochytrium.

3. The feed composition of claim 1, wherein the biomass derived from microalgae comprises at least one selected from a group consisting of the microalgae, a culture of the microalgae, a dried product of the culture, and a pulverized product of the dried product.

4. The feed composition of claim 1, wherein the fish of family Salmonidae is at least one selected from a group consisting of genus of Coregonus, genus of Prosopium, genus of Stenodus, genus of Thymallus, genus of Salvelinus, genus of Oncorhynchus, genus of Brachymystax, genus of Hucho, genus of Parahucho, genus of Salmo, and genus of Salvethymus.

5. The feed composition of claim 1, wherein the fish of family Salmonidae is Atlantic salmon (*Salmo salar).*

6. The feed composition of claim 1, wherein the biomass derived from microalgae comprises 50 to 90 wt.% of protein.

7. The feed composition of claim 6, comprising the biomass derived from microalgae of 1 to 100 wt.%.

8. The feed composition of claim 1, wherein nutrient digestibility of phosphorus is 40 to 60%.

9. The feed composition of claim 1, wherein nutrient digestibility of ash is 26 to 40%.

10. The feed composition of claim 1, wherein the composition is in a form of a mixed feed (Extruded pellet) or a raw feed (Moist pellet).

11. A method of manufacturing the feed composition for fish of family Salmonidae of any of claims 1 to 10.

12. A method of aquaculture for fish of family Salmonidae comprising a step of feeding a biomass derived from microalgae or the feed composition for fish of family Salmonidae of any one of claims 1 to 10 to the fish of family Salmonidae.

13. A method of improving digestibility of fish of family Salmonidae comprising a step of feeding a biomass derived from microalgae or the feed composition for fish of family Salmonidae of any one of claims 1 to 10 to the fish of family Salmonidae.

14. A use of improving digestibility of fish of family Salmonidae of a biomass derived from microalgae or the feed composition for fish of family Salmonidae of any of claims 1 to 10.

15. A composition for improving digestibility of fish of family Salmonidae, comprising a biomass derived from microalgae or the feed composition for fish of family Salmonidae of any one of claims 1 to 10.
